Europäisches Patentamt

European Patent Office

Office européen des brevets

(11) Publication number: **0 244 760**
A2

# EUROPEAN PATENT APPLICATION

(21) Application number: **87106271.7**

(22) Date of filing: **30.04.87**

(51) Int. Cl.⁴: **A 61 K 39/245**, A 61 K 39/255

(30) Priority: **01.05.86 US 858604**

(43) Date of publication of application: **11.11.87**
**Bulletin 87/46**

(84) Designated Contracting States: **AT BE CH DE ES FR GB GR IT LI LU NL SE**

(71) Applicant: **Salsbury Laboratories, Inc., 2000 Rockford Road, Charles City Iowa 50616 (US)**

(72) Inventor: **Sevoian, Martin, 167 Montague Road, North Amherst Massachusetts 01059 (US)**

(74) Representative: **Vossius & Partner, Siebertstrasse 4 P.O. Box 86 07 67, D-8000 München 86 (DE)**

(54) Vaccine for the prevention of Marek's disease.

(57) A cell free supernatant of whole, live cells of an attenuated lymphoblastoid cell line has been isolated which comprises an immunogenic agent which provides protection to an animal against infection by Marek's disease virus or a serologically related strain thereof when contacted with the immune system of the chicken. The cell line JMV-1 (ATCC No. CRL 9093) is utilized. A vaccine for conferring upon a chicken active immunity against infection by Marek's disease virus or a serologically related strain thereof comprises per dose an effective immunizing amount of cell free supernatant and suitable carrier.

# VOSSIUS & PARTNER 0244760

### PATENTANWÄLTE
### EUROPEAN PATENT ATTORNEYS

Dr. VOLKER VOSSIUS, Dipl.-Chem.
DOROTHEA VOSSIUS, Dipl.-Chem.
Dr. PAUL TAUCHNER, Dipl.-Chem.
Dr. DIETER HEUNEMANN, Dipl.-Phys.
Dr. PETER RAUH, Dipl.-Chem.
Dr. GERHARD HERMANN, Dipl.-Phys.

SIEBERTSTRASSE 4
P.O. BOX 860767
8000 MÜNCHEN 86
PHONE: (089) 474075
CABLE: BENZOLPATENT MÜNCHEN
TELEX: 529453 VOPAT D
TELEFAX: (089) 472001 (GR. II + III)

u.Z: W 469 EP
Case: 23998-A-EPO
Salsbury Laboratories, Inc.

2 3. APR. 1987

## VACCINE FOR THE PREVENTION OF MAREK'S DISEASE

Throughout this application, various publications are referenced by the name of the author and date of publication within parentheses. Full citations for these references may be found at the end of the specification listed in alphabetical order immediately preceding the claims. The disclosures of these publications in their entireties are hereby incorporated by reference into this application in order to more fully describe the state of the art as known to those skilled therein as of the date of the invention described and claimed herein.

Marek's disease is a naturally-occurring, malignant lymphoproliferative disease of chickens. It is caused by a cell-associated herpesvirus that is highly contagious and that readily spreads to susceptible chickens.

The disease was first described by Josef Marek in 1907 as a polyneuritis, which reflects the characteristic development of inflammatory nerve lesions in affected birds. Workers subsequently identified the development of lymphomas in nerves, visceral organs, muscle, and skin, and established the neoplastic nature of the disease.

Although many cell types may be infected with Marek's disease virus (MDV), the target for transformation is the T cell (Powell et al., 1976). The virus initiates a vigorous humoral and cellular immune response in the host against both viral and tumor cell antigens. While the ultimate outcome of the disease is the development of gross lymphomas, there is an early, severe depletion of lymphocytes in the primary lymphoid organs of the spleen, thymus and bursa of Fabricius. This depletion is due to a lytic infection of the B and T cells within these organs and in susceptible chicks, leads to a severe immunosuppression of the immune response (Calnek, 1980). Chicks which are resistant to Marek's disease can recover from an initial immune suppression to become persistantly infected with MDV for the rest of their lives. This chronic viremia stimulates a good serum neutralizing (SN) antibody titer and this SN antibody can be passed through the yolk sac to provide protection of progeny against MDV for 3 to 4 weeks post-hatch (Chubb and Churchill, 1968).

Marek's disease has been the cause of great economic losses in the past and continues to plague the poultry industry in spite of the successful development of vaccines in the early 1970's (Witter et al., 1970, Okazaki, 1970, Rispens et al., 1972). These vaccines represented the first effective and practical control of any cancer, virally or otherwise, in any species.

Apathogenic and attenuated strains of MDV and the apathogenic herpesvirus of turkey (HVT) have been widely used for protection against oncogenic MDV. HVT has been shown to preserve the cell-mediated immune response in vaccinates (Schierman et al., 1976) and all of these strains apparently protect against acute infection by avoiding the early cytolytic infection of the primary lymphoid organs (Calnek et al., 1979).

However, although they decrease infection somewhat, none of these isolates prevent super infection with oncogenic MDV and more virulent strains of the virus are surfacing. Vaccine breakthroughs have led to the current use of multivalent vaccines (Witter and Lee, 1984).

Many serologically related forms of Marek's disease virus (MDV) have been isolated, the first of which was the JM strain, isolated by Sevoian in 1962. Sevoian passaged MDV-infected blood through susceptible chicks, which resulted in the establishment of the lethal lymphoblastic leukemia designated JMV (Sevoian, 1967). In initial studies to determine the size of the JMV "agent", Hong (1973) filled diffusion chamber rings, which were sealed with Millipore membranes of various porosities, with JMV blood from moribund chicks and implanted the rings into the peritoneal cavity of day-old chicks. While mortality of chicks that carried a filter of 0.22 microns or greater indicated that a filterable agent or virus was involved, no MDV was ever recovered from kidneys of the test chicks nor could cytopathic effects (CPE) characteristic of MDV infection be demonstrated by cocultivation of JMV blood with permissive cells.

The resulting controversy over whether JMV tumors arise from de novo viral transformation of host lymphocytes or from growth of transplanted JMV cells within the host was resolved when JMV was shown to be a tumor transplant (Stephens et al., 1976). JMV lymphoblastoid cell lines have since been established as in vitro suspension cultures (Hahn et al., 1977, Munch and Sevoian, 1977, Nazerian et al., 1977). All express a female karyotype, carry a Marek's disease-associated, tumor specific antigen (MATSA), and express T cell surface markers. The line established by Hahn et al., (1977) (JM-VLC) is unique from the other two in that initially it appeared to carry MDV antigens. Virus could be identified in the kidney cells of chicks inoculated with this line by fluorescent and antibody assay and antibodies against MDV could be elicited in chicks that had been inoculated with JM-VLC. Conversely, the JMV-1 cell line established by Munch and Sevoian (1977) and the MDCC-RP1 (formerly RPL-1 line of Nazerian et al., (1977)) have been shown to be free of any serologically detectable or inducible virus. Several MDV genome copies are known to be present in each line although they may be incomplete, thereby preventing expression of the virus.

There are several practical Marek's disease vaccine types currently in use. These include attenuated oncogenic MDV (Churchill et al., 1969, Rispens et al., 1972), naturally apathogenic HVT (Kawamura et al., 1969, Witter et al., 1970, Okazaki, 1970), naturally non-oncogenic or apathogenic MDV (Rispens et al., 1972), or a combination of these in new polyvalent vaccines (Witter and Lee, 1984). The herpesvirus of turkeys (HVT), which is the most widely used commercial vaccine against Marek's disease, has antigenic properties that are quite similar to the herpesvirus of Marek's disease.

Many experimental vaccines composed of subcellular or whole cells preparations bearing virus or tumor antigens have contributed significantly to the understanding of the mechanisms of protection in Marek's disease (Lesnik and Ross, 1975, Kaaden et al., 1974, Powell, 1975).

Most notable is the transplantable, lethal lymphoblastic leukemia, JMV, which was developed from the rapid passage of the JM strain of MDV through susceptible chickens (Sevoian, 1967). This rapid passage resulted in a reduced incubation period and increased mortality and the JMV strain has since been used effectively as a challenge strain in vaccination studies against MDV (Hong and Sevoian, 1974, Shieh and Sevoian, 1974, Shieh and Sevoian, 1975, Munch and Sevoian, 1980) and has been developed as an *in vitro* cell line (Nazerian et al., 1977, Hahn et al., 1977, Munch and Sevoian, 1977).

In 1974, Shieh and Sevoian showed that JMV was more antigenically related to, if not identical with, JM than with HVT. They found that JMV-immunized chicks developed significantly higher levels of serum neutralizing antibody than did either JM or HVT-exposed chicks. These antibody levels were found to drop after 4 to 5 weeks. Hong and Sevoian, in 1974, looked at maternal antibody development associated with JMV immunization. They found that serum neutralizing antibody was elicited in response to JMV immunization in both the vaccinated dams and in their progeny. Further, progeny of immunized dams had a significantly higher protection against JM virus infection and tumor development than progeny of either JM- or HVT-immunized dams. The progeny of JMV-immunized dams which were naturally exposed to JM for 3 weeks yielded only 12% virus recovery from cultured kidneys as compared with 100% virus recovery from progeny kidneys from either HVT-immunized or non-immunized dams. Landgraf and Vielitz (1978) were able to demonstrate complete protection against JMV with attenuated JMV-A but only a 40-60% protection against MDV. No protection was found in progeny of hyperimmunized dams.

In 1977, Munch and Sevoian were able to establish an in vitro cell line from spleens of JMV-infected chicks, which was designated JMV-1. In vitro passage of JMV-1 cell produced changes in many cell properties, the most dramatic of which was a decrease in lethality. Early titrations indicated lethal-dose-fifty-percent (LD 50) values of 100 cells while later passages required LD 50's of $2 \times 10^5$ cells or greater. The decrease in lethality was not accompanied by a decline in antigenicity. Chicks which were vaccinated with late-passage attenuated JMV-1 cells survived a subsequent challenge with the more virulent wild type JMV (Munch et al., 1978). Spleen cells from birds resistant or susceptible to Marek's disease and immunized with JMV-1 prior to MDV challenge were found to be more cytotoxic to challenge tumor cells than were non-immunized controls. Non-immunized, but MDV-infected birds showed a negative cytotoxic response indicative of immune supression. Glutaraldehyde fixed or membrane extracts of JMV-1 cells failed to provide protection against wild type JMV challenge.

U.S. Patent No. 3, 981,771 (Sevoian) discloses a vaccine for immunizing poultry against Marek's disease virus and processes for producing the vaccine. The vaccine is obtained by modifying the JM strain of Marek's disease by rapid serial passage through selected tissue of live chickens until highly virulent followed by attenuation thereof in avian embryos.

Although several genome copies of MDV have been demonstrated in the cells, the line is non-productive of any virus and no viral proteins are serologically detectable in the cells. No virus can be recovered from kidneys of JMV-1-inoculated chicks or from co-cultivation of JMV-1 cells with chick embryo fibroblasts (CEF). Neither is virus inducible from the cells by treatment with 5-Bromo-2'-deoxyuridine (BRDU). Nevertheless, in 1980, Munch and Sevoian reported that immunization of day old chicks with attenuated JMV-1 cells provided them with a significant reduction in tumor development and recoverable virus from kidneys when compared with non-immunized, MDV-challenged chicks. The levels of precipitating antibody were significantly enhanced in the JMV-1-immunized, MDV-challenged chicks as compared with non-immunized, MDV-challenged chicks.

Nazerian and Witter (1984) were unable to show similar protection against MDV infection and tumor and development in a recent immunization trial with the non-productive MDCC-RP1 line. Von Bulow (1979) has previously shown that culture supernatant from the RP1 line was unable to protect against JMV challenge.

The present invention concerns the use of a cell-free, culture supernatant from the non-productive JMV-1 cell line for immunization against lethal JMV and against Marek's disease virus (MDV) challenge. JMV-1 cell-free supernatant was able to protect chicks against challenge with lethal JMV as well as, or better than, live JMV-1 cells.

Currently used Marek's disease vaccines are prepared from live type 2 or 3 Marek's disease herpesvirus which are most effective as cell-associated vaccines. These vaccines must be stored in liquid nitrogen.

The vaccine of the present invention is prepared from the spent media used in the growth of the JMV-1 tumor cell line. The spent growth media or supernatant does not contain viable virus or JMV-1 cells. Since the supernatant is nonviable, storage, transportation and handling will be considerably easier than needed for either cell associated frozen or cell-free lyophilized virus vaccines.

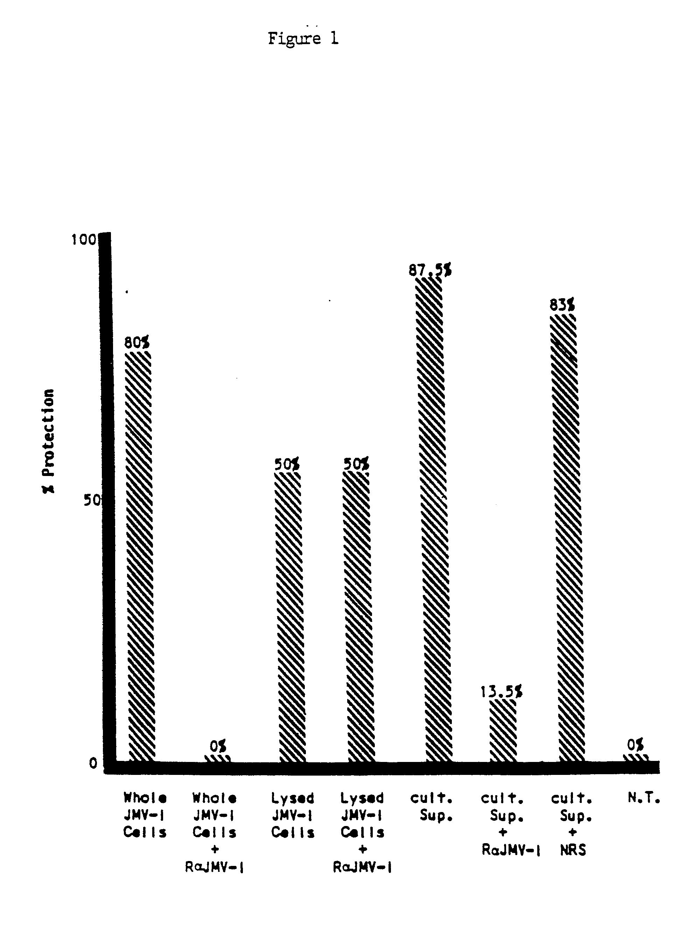

Figure 1 depicts the percentage of chickens protected against transplantable JMV by attentuated JMV-1 cells and JMV-1 culture supernatant before and after pretreatment of immunizing material with normal (NRS) or immune (R-alpha-JMV-1) rabbit serum.

The present invention provides a cell free supernatant of whole, live cells of an attenuated lymphoblastoid cell line which provides protection to an animal against infection by a herpesvirus or a serologically related strain thereof. The herpesvirus may be an avian herpesvirus, particularly Marek's disease virus or a strain serologically related to Marek's disease virus, preferably the lymphoblastoid cell line JMV-1 (ATCC No. CRL 9093).

This invention also provides a vaccine for conferring upon an animal active immunity against infection by a herpesvirus or a serologically related strain thereof which comprises per dose an effective immunizing amount of the cell free supernatant of the present invention and a suitable carrier. The animal is preferably avian, and more preferably a chicken.

In one embodiment of this invention, a vaccine for conferring upon a chicken active immunity against infection by Marek's disease virus or a serologically related strain thereof comprises per dose an effective immunizing amount of a cell free supernatant of the attenuated lymphoblastoid cell line JMV-1 (ATCC No. CRL 9093) and a suitable carrier.

The invention further provides a method of preparing the supernatant which comprises:

a. centrifuging a culture of whole live cells of a lymphoblastoid cell line derived from an animal infected with a herpesvirus or a serologically related stain thereof, so as to separate the cells from the supernatant;

b. treating the product of step (a) so as to remove the cells; and

c. recovering the supernatant.

The invention also relates to the above mentioned vaccine for use in protecting an animal against infection by a herpesvirus or a serologically related strain thereof.

Finally, the invention relates to the cell free supernatant for use in conferring upon a chicken active immunity against infection by Marek's desease virus or a serologically related strain thereof.

A cell free supernatant of cultures of whole, live cells of an attenuated lymphoblastoid cell line provides protection to an animal against infection by a herpesvirus or a serologically related strain thereof. The herpesvirus may be an avian herpesvirus, particularly Marek's disease virus or herpes virus of turkeys. In one embodiment the strain is serologically related to Marek's disease virus. A preferred strain is an attenuated lymphoblastoid cell line designated JMV-1 (ATCC No. CRL 9093). Preferably, the animal is avian, and more preferably the animal is a chicken or a turkey. However, the present invention also provides a cell free supernatant which provides protection to other animals against infection by a herpesvirus or a serologically related strain thereof.

Strain JMV-1 (ATCC No. CRL 9093) has been deposited with the American Type Culture Collection, Rockville, MD. This deposit was made pursuant to The Budapest Treaty On The International Recognition Of The Deposit Of Microorganisms For The Purposes Of Patent Procedure.

A vaccine for conferring upon an animal active immunity against infection by a herpesvirus or a serologically related strain thereof comprises per dose an effective immunizing amount of the cell free supernatant of the present invention and a suitable carrier. The herpesvirus may be any avian herpesvirus. Preferably, the herpesvirus is Marek's disease virus or herpes virus of turkeys or a strain serologically related to Marek's disease virus or herpesvirus of turkeys. The presently preferred strain is an attenuated

- 13 -

0244760

lymphoblastoid cell line designated JMV-1 (ATCC No. CRL 9093). In one embodiment of this invention the animal is avian, a chicken or a turkey. However, the present invention also provides a vaccine to provide protection to other animals against infection by a herpesvirus. Thus, a vaccine for conferring upon a chicken active immunity against infection by Marek's disease virus or a serologically related strain thereof comprises per dose an effective immunizing amount of a cell free supernatant of the attenuated lymphoblastoid cell line designated JMV-1 (ATCC No. CRL 9093) and a suitable carrier.

This invention further provides a method of preparing the supernatant which comprises:

a.  centrifuging a culture of whole live cells of a lymphoblastoid cell line derived from an animal infected with a herpesvirus or a serologically related strain thereof, so as to separate the cells from the supernatant;

b.  treating, e.g. filtering the product of step (a) so as to remove the cells; and

c.  recovering the supernatant.

The use of the supernatent for conferring upon a chicken active immunity against infection by Marek's disease virus or a serologically related strain thereof comprises administering to a chicken an effective immunizing amount of the supernatant of the present invention. An effective immunizing amount is typically above about 0.2 ml. of the supernatant.

The use of the vaccine for protecting an animal, e.g. a chicken, against infection by a herpesvirus or a serologically related strain thereof, e.g. Marek's disease virus, comprises administering to the animal a suitable dose of a vaccine according to the present invention.

The cell free supernatant of the present invention may be administered to chickens by any of a number of well known methods. Desirably, the administration may involve intraperitoneal injection or subcutaneous or intramuscular injection at the back of the neck. If the mode of administration involves injection, any pharmaceutically acceptable carrier may be employed. Examples of suitable carriers include 0.01 to 0.1M, e.g. 0.05M, phosphate buffer or 0.8 percent saline.

In addition, the carrier desirably contains a preservative. One particularly suitable preservative is thimerosal (sodium ethylmercurithiosalicylate) which has activity as both a bacteriostat and a fungistat. Desirably, thimerosal is present in the vaccine in a final concentration of about $10^{-4}$ percent.

Finally, the carrier desirably also contains an immunopotentiator or adjuvant. Various immunopotentiators known in the art may be used. A suitable adjuvant is 94% Drakeol 6-VR, 5% Arlacel A, 1% Tween-80. Arlacel A is a mannide monoleate (Sandria Corp.). It is an irritant which has strong immunopotentiating activity when combined with antigens. Drakeol 6-VR is a hypoallergenic light mineral oil product (Penreco Corp.). Tween-80 is a monoleate derivative of polyoxyethylsorbitan and possesses detergent properties. Other suitable carriers or immunopotentiators include aluminum potassium sulfate, aluminum hydroxide, lymphokines and water in oil emulsions.

Studies were undertaken to demonstrate _in vivo_ protection against Marek's disease by immunization with supernatant derived from the JMV-1 cell line. Protection assays against lethal JMV demonstrated that $1 \times 10^2$ whole, live JMV-1 cells and cell-free culture supernatant from as few as $1 \times 10^4$ JMV-1 cells could provide up to 100% protection against lethal JMV challenge. This protective activity could be blocked by pretreatment of JMV-1 cells or culture supernatant with rabbit antiserum produced against JMV-1 cells and absorbed to specificity with normal chicken tissue. Normal rabbit serum, similarly absorbed, did not block this protection. The protection provided by cell-free supernatant could be diluted out at $1 \times 10^4$ cells.

Protection assays against the highly oncogenic Conn B strain of MDV showed that a cell-free immunization with $1 \times 10^6$ JMV-1 cells elicited a significant reduction in virus recovery from cultured kidney cells and in lymphoma development in P and N line chicks as well as in SPAFAS and broiler stock. This protection began to decrease by 7 weeks post-MDV challenge in P line chicks but remained protective in the other 3 lines of chicks through 7 weeks post-MDV challenge.

Assays of cell mediated and humoral immunity were conducted to determine the effect of immunization with JMV-1 cell antigens on the immune response of the host.

In the DTH assay of cell and mediated immunity, P line chicks immunized with JMV-1 cells retained their DTH response to L. monocytogenes after challenge with MDV but N, K, and S line chicks did not. The DTH response of P line chicks did not correlate with resistance to MDV or tumor development. In vitro results from the $^{51}$Cr release assay and the plaque inhibition assay indicated that no cell mediated response was generated against tumor cell antigens or viral antigens in cell-free supernatant immunized chicks before or after challenge with MDV.

The virus neutralization assay results indicated that a significant enhancement of the neutralizing antibody response against MDV was elicited in chicks which had been immunized with cell-free JMV-1 culture supernatant prior to challenge with MDV. No neutralizing or precipitating antibody response was elicited in supernatant-immunized chicks in the absence of MDV challenge. Neither was any maternal antibody detected in progeny or supernatant-hyperimmunized dams.

The data suggest that chicks are protected against virus infection by immunization with cell-free JMV-1 culture supernatant because MDV is neutralized by an enhanced antibody response and that lymphoma development is reduced as a consequence of decreased viral infectivity.

The experimental data which follow demonstrates that antigen(s) which are protective against MDV and against the lethal transplant, JMV, are shed into the culture supernatant from the JMV-1 cell line and these antigens retain their immunogenicity. Virus neutralization assays demonstrate an enhancement of the anitbody response against MDV in supernatant-immunized, MDV-challanged chickens.

While no MDV antigens are serologically detectable or inducible from the JMV-1 cell line (21), there is clearly an antigenic relationship between JMV-1 and MDV and HVT. The protection against JMV afforded by HVT and SB-1, however, supports the possibility that JMV-1 cells express MDV antigens which are not only undetectable by ordinary serological techniques, but also are probably present in such minute quantities as to provide an undetectably low level of initial immune stimulation, which then appears to be extra-ordinarily enhanced upon MDV challenge. This may account for the observed virus neutralization titers (Table 14). Nonetheless, one cannot rule out the possibility that the protection that has been seen against both MDV and JMV by the culture supernatant could be the result of the presence of both specific antigens and non-specific modulators effecting the observed immune response. Further studies will have to be conducted with both herpesviruses and non-herpesviruses, as well as with MDV-related and non-MDV-related tumor cell lines, in order to clarify the specificity of the immune response.

With the discovery of the presence of protective antigens in the culture supernatant the opportunity is provided to explore the immune response of chicks to MDV protective antigens without the direct influence of viral antigens or tumor cells. In addition, the protective nature of the supernatant permits the development of a non-oncogenic and non-infectious immunizing agent against Marek's disease.

The JMV-1 supernatant enhances both the active and passive serological response of chickens to type 1 Marek's disease virus. This enhancement may permit greater maternal antibody protecton of progeny from dams receiving the supernatant alone or in combination with live Marek's disease vaccines such as HVT, SB-1 or CVI 988 clone C (Rispen).

## Experimental Details

## Materials

**Experimental animals.** Specific pathogen-free chickens were obtained by incubation of eggs from SPAFAS, Inc., Norwalk, CT and from Line N and P eggs from Dr. B. W. Calnek, Cornell University, Ithica, N.Y. N and P line chicks, homozygous at the B 21 and B 19 major histocompatibility locus, respectively, have been bred for their respective resistance or susceptibility to Marek's disease (Hutt and Cole, 1947). A cornish/White Rock cross of stock meat chickens was obtained by incubation of eggs from Hubbard Farms, Walpole, NH. K and S line chicks were obtained by incubation of eggs from the Regional Poultry Labs. East Lansing, MI K line and S line chickens are bred for their respective resistance and susceptibility to Marek's disease. K line chicks are homozygous at the B 15 allele and S lines segregate for 3 possible combinations of B 1 and B 15 (Pazderka et al., 1975). All chicks were maintained in Horstall Bauer isolation units.

**Viruses.** The Conn B strain or MDV was passaged through P line chicks and whole, heparinized blood was withdrawn from chicks showing clinical signs of Marek's disease. MDV-infected blood was titered on chick kidney cells and known quantities of plaque-forming units were stored in liquid nitrogen for use as challenge material.

Cell-free MDV was obtained by removing the feather tips and skin from the main feather tracts of Conn B-infected chickens, 2 to 6 wks p.i. This material was placed in stabilizing buffer (0.218 M Sucrose, 0.00038M $KH_2PO_4$, 0.0072M $K_2HPO_4$, 0.0049M MSG, 1% BSA, 0.2% EDTA.) after Calnek et al., (1970), minced with scissors and homogenized in a Virtis mixer for 2 mins., on ice. The supernatant was separated from remaining feather and skin by filtration through gauze and the supernatant was freeze-thawed by immersion in liquid $N_2$ and thawing in tap $H_2O$, 3 times, then sonicated, on ice, for 2 mins. in a Biosonic Oscillator at 60 kcyl/sec. Supernatant was centrifuged at 10,000 X g for 10 mins., then filter-sterilized through a 0.4 micron filter which had been presoaked in FCS. Aliquots of cell-free virus were titered on chick kidney cells and frozen in 10% DMSO in liquid $N_2$.

L. monocytogenes antigen isolation. Listeria monocytogenes, strain A4413 was provided by Dr. Martin S. Wilder of the Department of Microbiology, University of Massachusetts. Cultures were stored at - 20°C on tryptose agar slants, and bacteria was grown in 200 mls of tryptose broth for 24 hrs. prior to use. Antigen was precipitated from culture with saturated $(NH_3)_4SO_4$ at 4°C, and the precipitate was centrifuged at 5000 X g for 15 mins. The resulting pellet was redissolved in 50 mls of PBS, ($Na_2HPO_4$, 3.44g; $KH_2PO_4$, 6.92 g; NaCl, 4.5g; distilled $H_2O$, 1000ml), pH 7.2, dialyzed against PBS for 48 hrs. and pervaporated to 3 to 5 mls. The concentrated antigen was purified on a Sephadex G-50 column, in PBS at pH 7.2 and the protein in the first peak off of the column was quantitated by the Lowry protein assay (Lowry et al., 1951).

The optimum amount of listeria antigen required for the delayed type hypersensitivity (DTH) assay was determined by inoculation of chicks with antigen dilutions of 5, 10 and 20 μg, in one footpad versus PBS in the other footpad, 3 days after sensitization with L. monocytogenes.

Cell cultures. The JMV-1 cell line has been maintained in continuous passage by diluting cultures every 48 hrs., 1:10 in RPM1 1640 media (GIBCO, Grand Island, NY), supplemented per liter with 1 mM sodium pyruvate, 2mM L-glutamine, 0.1 mM MEM non-essential amino acids, 5% tryptose phosphate broth, $10^{-3}$ mM B-mercaptoethanol, 10% heat-inactivated chicken serum (CS), 8% fetal calf serum (FCS) and penicillin, dihydrostreptomycin and mycostatin at 10,000 units, 10,000 micrograms and 2500 units, respectively. Cultures were incubated at 37°C in a 5% $CO_2$ in air atmosphere.

JMV-1 cell-free supernatant was prepared from 48 hour cultures prior to passage. Viable cell counts were made by trypan blue exclusion. Cultures were centrifuged at 4000 X g for 5 minutes and the resulting culture supernatant was filtered through a 0.4 micron porosity nitrocellulose membrane.

The lethal transplant, JMV, was maintained via in vivo passage through N line chickens, as previously described. Liver and spleen homogenates from moribund chicks were titered in day-old chicks and aliquots of 10,000 chick lethal doses (CLD) were stored in liquid nitrogen for use as challenge material.

Chick kidney cell monolayers were established from chicks of various treatment groups which had been ex-sanguinated by cardiac puncture. Kidneys were asceptically harvested and connective tissue was removed. The tissue was gently pushed through a syringe into a 0.25% trypsin in phosphate buffered saline (PBS) solution, pH 7.2. Cell suspensions were trypsinized for 10 minutes, 3 times, washed 3 times in Media 199 (GIBCO, Grand Island, NY) supplemented per liter with 0.6% sodium bicarbonate, 10% tryptose phosphate, antibiotics, as above, and 1% FCS, and cultured at 37°C in a 5% $CO_2$ in air atmosphere. MDV plaques were enumerated after 7 to 10 days of culture.

Polyclonal antibody production. Young adult, female, New Zealand rabbits were inoculated 4 times via the peripheral ear vein over a 4 week period, with increasing doses of JMV-1 cells, suspended in PBS. Rabbits were ear-bled, 7, 17 and 27 days after the last inoculation and the serum from whole blood was inactivated at 56°C for 30 mins., and then absorbed with CRBCs, chick-Ig, liver, spleen, thymus and bursal cells. Specificity of the antisera for JMV-1 cells was determined by fluorescent antibody assay. Cell suspensions of 1 X $10^6$ thymus or bursa cells or JMV-1 cells were incubated in 50 microliters of JMV-1 antiserum or control rabbit serum for 20 mins. at 4°C. Cells were washed three times in PBS and resuspended in goat anti-rabbit sera conjugated to fluorescein isothiocyanate (FITC), incubated for an additional 20 mins. as before and washed three times in PBS. Cells were resuspended in ice cold 70% glycerol in PBS and examined immediately under ultraviolet light on a standard Zeiss UV-light microscope.

## Methods

Protection against lethal, transplantable JMV. Immunization trials in day-old N line chicks were conducted with 1 X $10^2$ whole, live JMV-1 cells, previously found to be protective against JMV challenge, or with 1 X $10^2$ JMV-1 cells which had been lysed in distilled water by ultrasonic treatment, or with culture supernatant, filtered as above, from $10^6$ JMV-1 cells, all from 48 hour cultures of JMV-1 cells. Fifteen days after immunization, chicks were challenged with 1000 CLD of JMV and observed for 15 days for JMV-specific mortality as determined by splenomegaly, hepatomegaly and death, 3 to 14 days after challenge.

These trials were repeated with treatments as above except that prior to immunization, JMV-1 cells and supernatant were incubated with a 1:80 dilution of rabbit-JMV-1 antisera or of normal rabbit serum, absorbed in parallel with the anti-JMV-1 sera, with normal chicken tissue, for 30 minutes at $37^{\circ}$C.

The lethal-dose-fifty-percent ($LD_{50}$) and protective-dose-fifty-percent ($PD_{50}$) of JMV-1 cells and cell-free supernatant was determined for challenge with lethal JMV by the method of Reed and Meunch (1938). Six groups of day-old chicks were inoculated interperitoneally (ip) with 10-fold serial dilutions of JMV-1 cells or with cell-free supernatant from 1 X $10^7$ JMV-1 cells from 48 hour cultures. Chicks were observed for JMV-specific mortality for 15 days, and then challenged with 1000 CLD of JMV and observed as above for JMV-specific mortality.

<u>Protection against MDV challenge</u>. Assays of protection by JMV-1 culture supernatant against MDV infection and tumor development were conducted in N line, P line, broiler and SPAFAS chickens. One half of the chicks in each trial were immunized ip, at one day-of-age with 0.2 ml of filtered culture supernatant from $1 \times 10^6$ JMV-1 cells. Fifteen days later, one half of each group was challenged with 200 - 500 plaque forming units (PFUs) of the Connecticut B strain of MDV (Jakowski et al., 1970). At 2, 5, and 7 weeks post-challenge, one third of each group was exsanquinated and examined for gross tumor development. Kidneys were cultured as above for virus recovery. Spleens were removed for use in a $^{51}$Cr release assay and serum samples were saved for virus neutralization studies.

Five trials each were conducted with N and P line chicks, 1 trial with broilers and 2 trials with SPAFAS chicks. In 2 of the 5 N and P line trials, cell-free supernatant was emulsified in a 1:1 mixture with a mineral oil-based adjuvant and injected sub-cutaneously at several sites as well as ip.

<u>DTH assay</u>. The effects of immunization with JMV-1 cells and subsequent MDV challenge on the cell-mediated DTH response of chicks was measured. Four treatment groups included; No-treatment controls, day-old chicks receiving $1 \times 10^2$ JMV-1 cells, day-old chicks receiving $1 \times 10^2$ cells and then MDV challenge 15 days later, and chicks receiving only a challenge dose of MDV at 15 days of age. Fourteen days after MDV challenge, all groups - with the exception of 3 birds from control group 1 - were inoculated, i.p., with 1 ml of a 1:10 dilution of 24-hr old listeria culture in PBS. Four days after sensitization with the <u>Listeria</u> culture, <u>Listeria</u> antigen in PBS was injected into one footpad and only PBS was injected into the other footpad of sensitized chicks and of non-sensitized control chicks. After 24 hrs., the degree of footpad swelling was measured by dial gauge calipers and the DTH index was determined as the degree of swelling in test chick footpads minus the degree of swelling in control chick footpads, all divided by the degree of swelling in the control chick footpads.

$^{51}$Cr <u>release assays</u>. Treatment groups were described previously in the section entitled Protection against MDV challenge. Sensitized lymphocytes were obtained from spleens, which were asceptically harvested and gently ground in Ten Broeck grinders in PBS. Particulate matter and red blood cells were allowed to settle for 30 mins and cell densities were adjusted to $5 \times 10^6$ viable cells/ml of RPMl 1640.

Target cells were obtained from 24 hr suspension cultures of JMV-1 and MSB-1 (Akiyama and Kato, 1974) cell cultures or chick kidney cell monolayers in 24 well tissue culture plates. Suspensions of $1 \times 10^7$ JMV-1 and MSB-1 cells in RPMl 1640 media were labelled with 200 micro-Curies of $^{51}$Cr in sterile saline (300-500 Cl/g S.A., New England Nuclear, Boston, MA). The mixtures were incubated for 2 hours at $37^{\circ}C$, then washed 5 times by centrifugation in RPMl 1640 containing 20% FCS. Cell density was adjusted to $1 \times 10^5$ viable cells/ml to give an effector to target cell ratio of 50:1.

Chick kidney cell monolayers were labelled with 200

micro-Curies of $^{51}$Cr/24 well plate and incubated for 2 hrs. at 37$^{\circ}$C, then washed at 5 times with PBS by rotation on a Tektator shaker. The effector to target cell ratio of these cultures was approximately 10:1.

Volumes of 200 microliters culture mixtures containing 100 microliters of target cells and 100 microliters of sensitized lymphoid cells were placed in a U-bottom, 96 well microtiter plate or 1 ml of sensitized spleen cells was added to each kidney cell well, and incubated in a 5% $CO_2$-in-air atmosphere for 6 hours, as previously optimized (Keller, 1981). The cultures were then centrifuged at 2000 rpm for 5 mins and 100 microliters of the supernatant fluid was removed and placed in scintillation vials in Scinti verse (Fisher Scientific) for counting in a Searle Delta 300 Liquid Scintillation System.

Total incorporation of label into target cells was determined by measurement or radioactivity of 3 samples of the reaction mixtures. Maximal $^{51}$Cr release was determined by incubating 100 microliters of reaction mixture with 2% NP-40 in 0.05 M Tris, pH 7.4, for 4 hrs. This was centrifuged at 2000 rpm for 5 mins and counted in Scinti verse. This activity represents 100% of releasable $^{51}$Cr and is identical to the spontaneous release of $^{51}$Cr from labelled target cells incubated without lymphoid cells. Results are expressed as percent lysis using the following formula: $^{51}$Cr release in the presence of immune lymphoid cells minus $^{51}$Cr released in the presence of normal lymphoid cells, all divided by maximal $^{51}$Cr released in the presence of normal lymphoid cells, all times 100 = % specific lysis.

Plaque inhibition assay. Effector cells were obtained from adult K line dams which had been given an initial inoculation of 3 mls of filtered supernatant from JMV-1 cultures, and 2 weeks later and against 12 weeks after that were given an additional inoculation of 2.0 mls of filtered culture supernatant. Leukocytes were separated from whole, heparinized blood by ficol hypaque cushion. Cells were washed 3 times in PBS and cell volumes were adjusted to $2 \times 10^5$, $2 \times 10^6$, and $2 \times 10^7$ cells/ml of 199 media supplemented with 10% FCS. Non-immunized K line hatchmates provided negative control PBLs which were prepared in the same manner.

Target cells were PBLs from 5 week-old K line chicks, infected with MDV at day of age. Leucocytes were isolated from whole, heparinized blood as above and adjusted to $2 \times 10^7$ cells/ml in 199 media supplemented with 10% FCS.

Increasing concentrations of sensitized effector cells in 0.5 mls were mixed with $1 \times 10^7$ target cells in 0.5 mls and the mixtures were overlaid onto nearly confluent monolayer kidney cell cultures and incubated at $37^{\circ}$C. Cultures were washed after 24 hours with 199 media supplemented with 10% FCS and observed for 2 weeks for plaque development. Plaques could usually be counted after 10 days of incubation.

Virus neutralization assay. Two-fold serial dilutions of a 1:20 dilution of test serum were mixed with 100 PFUs of cell-free virus and incubated at $37^{\circ}$C for 30 mins. The mixture was then overlaid onto chick kidney cell monolayers in 24 well tissue culture plates for 1 hr., at which time 1.0 ml of media 199, supplemented with 10% FCS, was added to each well. Dilutions were plated in duplicate and observed for plaque development for up to 14 days. Known positive and negative sera were included in each trial. The neutralizing antibody titer was considered as that dilution of serum which caused a 90% reduction in virus-induced plaques over infected cultures treated with sera which was negative for MDV.

Maternal antibody development. Chicks from hyperimmunized K line dams and from unimmunized K line hatchmates, were challenged at 1 day-of-age with approximately 500 PFUs of Conn E strain of MDV and at 2 weeks of age, were exsanquinated by cardiac puncture. The serum was saved for agar gel immunodiffusion assays (AGID) and kidneys were asceptically removed and established in culture as previously described. The cultures were observed for the development of MDV-specific cytopathic effects (CPE) for up.to 14 days.

Agar gel immunodiffusion test (AGID). The AGID test was used for the detection of MDV-specific precipitating antibody in serum samples, (Chubb and Churchill, 1969), using a 1% suspension of noble agar in PBS, pH 7.4, (Okazaki et al., 1970). Plates were incubated at room temperature for 72 hrs and observed for the development of precipitin lines of antigen/antibody reaction.

Field Challenge System.

Marek's disease challenge systems are well established. There are 3 primary challenge agents used for efficacy testing. The first is the JMV agent which is very virulent. Challenge with JMV results in mortality as high as 100 percent within 10 days. The second agent is the JM virus. The virus along with the Ga strain is recognized as the standard challenge. Thirty (30) to 50 percent mortality can be observed. These viruses produce primarily neural lesions with some visceral lesions. The third agent is the very virulent MD virus represented by RBIB. HVT or SB-1 MD vaccines when used will protect a maximum of 50 percent of the vaccinates. The RBIB may produce 80 percent or more mortality with primarily visceral lesions. All three of these agents may be used in evaluation of the JMV-1 supernatant vaccine.

## Establishment of Master Seed Passages

Since the active agent of the supernatant vaccine is nonviable, the JMV-1 cell line will be prepared as master seed. Loss of antigenicity due to continuous passage has not been reported. However, to insure consistency of the vaccine, passage will be limited to 20. The X passage will be established at the third consecutive passage with a viable cell count of about $10^{6.5}-10^7$ per ml. X+1 through X+19 will be utilized as working and production seed. The X, X+5, X+15 and X+20 passages will be used for karyology. Antigens present in the supernatant will be identified.

## EXAMPLE 1

In Vivo Protection Assays.

A.    Protection against lethal JMV by vaccination with JMV-1 cell line antigens. Immunization trials with 1 X $10^2$ whole, live JMV-1 cells or lysed JMV-1 cells, or with the supernatant, which had been filtered through a 0.4 micron filter, from $10^6$ JMV-1 cells, showed that cell-free supernatant protected chicks as well as or better than live JMV-1 cells when chicks were challenged with 1000 CLD of JMV (Table 1). These data also showed that viable JMV-1 cells are required for significant protection against challenge although some protection is afforded by lysed cells, and suggested that some of the antigens, which are protective against JMV challenge, are shed after production by JMV-1 cells in vitro.

This trial was repeated with treatments as above except that prior to immunization, JMV-1 cells and supernatant were incubated with a 1:80 dilution of rabbit anti-JMV-1 antisera or with normal rabbit serum, either unabsorbed or absorbed in parallel with immune serum, for 30 minutes at 37$^o$C (Table 2). Figure 1 demonstrates that treatment of the immunizing agents with rabbit anti-JMV-1 completely blocked the protective antigens on whole JMV-1 cells and significantly altered protection afforded by supernatant but did not change the protective character of lysed JMV-1 cells. Pretreatment of JMV-1 culture supernatant with normal rabbit serum did not effect the protection profile.

## TABLE 1

### JMV-specific mortality in immunized chicks.[a]

| Whole Cells | Lysed Cells | Supernatant | N.T. Control |
|---|---|---|---|
| 2/10[b] | 5/10 | 0/9 | 8/9 |
| 4/10 | 6/10 | 1/9 | 8/10 |
| 0/4 | 2/5 | 2/9 | 8/9 |

[a]Day-old chicks received $10^2$ live JMV-1 cells, $10^2$ lysed JMV-1 cells or the supernatant from $10^6$ cells which had been filtered through a 0.4 micron filter, or no treatment (N.T.). At 15 days-of-age, chicks were challenged with 1000 Chick Lethal doses (CDL) of JMV.

[b]JMV-specific mortality / number of chicks tested.

---

## TABLE 2

| Whole Cells (anti-JMV-1) | Lysed Cells (anti-JMV-1) | Supernat (anti-JMV-1) | Supernat (NRS) | Supernat (N.T.) | N.T. Ctrl |
|---|---|---|---|---|---|
| 10/10[b] | 5/10 | 9/10 | -- | -- | 9/9 |
| -- | -- | 5/6 | 1/6 | 1/6 | 6/6 |

[a]Day-old chicks received $10^2$ live JMV-1 cells, $10^2$ lysed JMV-1 cells or the supernatant from $10^6$ JMV-1 cells which had been filtered through a 0.4 micron filter, or no treatment (N.T.). Prior to immunization, all agents were incubated with a 1:80 dilution of JMV-1 rabbit antiserum (anti-JMV-1) or normal rabbit serum (NRS). At 15 days-of-age, chicks were challenged with 1000 CLD of JMV.

[b]JMV-specific mortality / number of chicks tested.

---

The $LD_{50}$ and $PD_{50}$ assays against lethal JMV have been conducted yearly with JMV-1 cells being maintained in continuous passage. Tables 3 and 4 show the results of these assays for JMV-1 cells from 1982 through 1984 and for cell free supernatant in 1984. JMV-1 cells have stabilized at an $LD_{50}$ of slightly less than 10,000 cells per chick while the cell-free supernatant is not lethal for chicks at any dilution of $1 \times 10^7$ JMV-1 cells.

All survivors of the initial immunization with JMV-1 cells have been found to be protected against lethal JMV challenge. However, the protection afforded by cell-free supernatant against lethal JMV is seen to dilute out at approximately 10,000 JMV-1 cells.

## TABLE 3

Lethal dose fifty percent of JMV-1 cells and cell free-supernatant.

|                      | Immunizing dose of JMV-1 cells or supernatant from same | | | | | | |
| -------------------- | ------ | ------ | ------ | ------ | ------ | ------ | ------ |
| Immunizing Agent     | $10^7$ | $10^6$ | $10^5$ | $10^4$ | $10^3$ | $10^2$ | $10^1$ |
| JMV-1, 1982          |        | 4/5[a] | 2/5    | 3/5    | 2/5    | 2/5    | 0/5    |
| JMV-1, 1983          |        | 3/6    | 4/6    | 3/6    | 1/6    | 0/6    | 1/6    |
| JMV-1, 1984          | 3/5    | 1/3    | 3/5    | 3/5    | 2/5    | 2/5    | 1/5    |
| Supernat, 1984       | 0/5    | 0/5    | 0/5    | 0/5    | 0/5    |        |        |

[a]Number of JMV-specific deaths/Number of chicks tested.

0244760

## TABLE 4

Protective dose fifty percent of JMV-1 cells and cell-free supernatant.

|  | Immunizing dose of JMV-1 cells or supernatant from same | | | | | | | |
|---|---|---|---|---|---|---|---|---|
| Immunizing Agent | $10^7$ | $10^6$ | $10^5$ | $10^4$ | $10^3$ | $10^2$ | $10^1$ | Cntrl |
| JMV-1, 1982 |  | 0/1[a] | 0/2 | 0/2 | 0/3 | 0/3 | 0/5 | 3/3 |
| JMV-1, 1983 |  | 0/3 | 0/2 | 0/3 | 0/5 | 0/6 | 0/5 | 5/5 |
| JMV-1, 1984 | 0/2 | 0/2 | 0/2 | 0/2 | 0/3 | 0/3 | 0/4 | 5/5 |
| Supernat, 1984 | 0/5 | 1/5 | 0/5 | 2/5 | 4/5 |  |  | 4/5 |

[a]Number of JMV-specific deaths/Number of chicks tested.

----------------------------------------------------------------

B.  **Protection against MDV-infection and tumor development by vaccination with JMV-1 culture supernatant.** Trials have been conducted with N and P line chicks, as well as with Hubbard broiler stock and pathogen-free (SPAFAS) chicks.  N and P line trials conducted with supernatant suspended in adjuvant yielded results that were not significantly different from non-adjuvant trials.  N and P line trials were run concurrently and non-specific losses of chicks due to equipment failures were high.  Therefore, the results expressed in Table 5 and in figures 2 and 3 are a compilation of the data obtained from 5 N line and P line trials.  Table 6 summarizes the results from 1 broiler trial and 2 SPAFAS trials.  These data indicate a highly significant reduction, ($P < 0.01$), in both virus recovery (Table 5 & 6) and gross tumor development (Table 7 & 8) in supernatant-immunized N and P line, broiler and SPAFAS chicks through 5 weeks post-MDV challenge.  After 5 weeks, mortality occurred among both immunized and non-immunized P line chicks although those that survived to 7 weeks showed low virus recovery. Immunization was able to protect N line, broiler and SPAFAS chicks against virus infection through 7 weeks (P<0.001).

---

## Table 5

Virus recovery[1] from N and P line chicks immunized with JMV-1 culture supernatant and challenged with MDV.

**P LINE**

| Treatment | 2 wks | 5 wks | 7 wks |
|---|---|---|---|
| Supernatant + MDV | $3.95 \pm 2.28^{2a}$ | $23.19 \pm 2.01^{b}$ | $121.67 \pm 2.69^{c}$ |
| MDV | $49.33 \pm 2.01^{d}$ | $58.11 \pm 2.46^{d}$ | $166.46 \pm 2.69^{c}$ |

**N LINE**

| Treatment | 2 wks | 5 wks | 7 wks |
|---|---|---|---|
| Supernatant + MDV | $3.09 \pm 1.82^{a}$ | $0.00 \pm 1.61^{a}$ | $22.96 \pm 2.01^{a}$ |
| MDV | $4.15 \pm 2.01^{a}$ | $84.38 \pm 2.13^{b}$ | $73.69 \pm 1.61^{b}$ |

[1] Day old chicks received immunizing dose of cell free JMV-1 culture supernatant or no treatment. At 15 days, 1/2 of each of these groups received 200-500 PFUs of Conn B strain of MDV. Assays conducted at 2, 5, and 7 weeks post-MDV challenge.

[2] Average number of plaques in $10^{6}$ kidney cells $\pm$ standard error of the mean of triplicate cultures. Plaque development was not observed in kidneys from chicks receiving no MDV challenge.

[a-d] Different superscripts indicate statistically significant differences between means within lines.

---

The least squares means of plaque development from immunized *versus* unimmunized chicks were compared by Student's t test. P line chicks which had been immunized with cell-free supernatant were found to be significantly protected against MDV-infection at 2 weeks (P < .05, 2.35, 49 df) and at 5 weeks (P < .05, 2.03, 48 df) post-MDV challenge, when compared with unimmunized chicks. At 7 weeks post-challenge, a significant increase in recoverable virus was noted in immunized and unimmunized P line chicks (P < .01, 3.28, 38 df). Within 2 weeks post-challenge, chicks challenged with MDV but not immunized with cell-free supernatant were producing high levels of recoverable virus. There was a wide range of response within given treatment groups. In unimmunized, but MDV-challenged chicks, plaque development of 200 or more per culture occured at 2, 5, and 7 weeks and ranged from 20% at 2 weeks to 80% at 7 weeks. At 2 weeks post-challenge, 50% of the remaining cultures yielded less than 10 plaques or none and 30% produced 10 - 50 plaques per culture. By 5 weeks, most cultures produced 20 - 80 plaques per culture. No cultures from immunized P line chicks at 2 and 5 weeks post-challenge produced more than 20 plaques with the exception of those from one chick assayed at 5 weeks post-challenge, which produced greater than 200 plaques per culture. Ninety percent of the cultures at 2 and 5 weeks post-challenge produced less than 5 plaques per culture or none. At 7 weeks post-challenge however, up to 80% of the cultures from both immunized and unimmunized chicks were producing more than 200 plaques per culture.

Kidney cell cultures from N line chicks at 2 weeks post-MDV challenge, showed no significant difference in plaque development between immunized and unimmunized

Table 6

Virus recovery[1] from broiler and Specific-pathogen-free chicks immunized with JMV-1 culture supernatant and challenged with MDV.

BROILERS

| Treatment | 2 wks | 5 wks | 7 wks |
|---|---|---|---|
| Supernatant + MDV | $0.00 \pm 2.69^{2a}$ | $2.14 \pm 2.69^{a}$ | $1.9 \pm 3.01^{a}$ |
| MDV | $0.00 \pm 2.46^{a}$ | $36.2 \pm 2.69^{b}$ | $200.0 \pm 4.26^{c}$ |

SPAFAS

| Treatment | 2 wks | 5 wks | 7 wks |
|---|---|---|---|
| Supernatant +MDV | $0.47 \pm 0.02^{a}$ | $0.10 \pm 0.02^{a}$ | $3.38 \pm 1.92^{a}$ |
| MDV | $3.81 \pm 0.85^{a}$ | $11.20 \pm 2.97^{b}$ | $47.92 \pm 5.14^{c}$ |

[1]Day old chicks received immunizing dose of cell free JMV-1 culture supernatant or no treatment. At 15 days, 1/2 of each of these groups received 200-500 PFUs of Conn B strain of MDV. Assays conducted at 2, 5, and 7 weeks post-MDV challenge.

[2]Average number of plaques in $10^{6}$ kidney cells $\pm$ standard error of the mean. Kidneys cultured in triplicate. Plaque development was not observed in kidneys from chicks receiving no treatment or supernatant immunization only.

[a-b]Different superscripts indicate statistically significant dirrerences between means within lines.

chicks. However, at 5 weeks post-challenge, a highly significant reduction (P < .001, 3.49, 49 df) in recoverable virus was observed from immunized versus unimmunized chicks and this reduction remained significant through 7 weeks post challenge (P < .01, 3.24, 48 df). Overall, cultures from unimmunized chicks produced significant (P < .05, 1.98, 67 df) levels of virally-induced plaques after 5 weeks post-challenge. At 5 weeks post-challenge, one third of the kidneys assayed developed no plaques, one third developed between 10 - 50 plaques per culture and one third developed over 200 plaques per culture. A similar pattern was seen at 7 weeks post-challenge. In immunized chicks however, no plaques were found in kidneys cultured at 5 weeks post-challenge and 7 weeks post-challenge, only one chick produced plaques of more than 200 per culture. The remaining cultures all yielded less than 4 plaques per culture or no plaques.

Virus recovery data from broiler stock and SPAFAS were less variable than those from N and P line trials and present a protection profile similar to that observed for N line chicks. Kidneys from broiler stock, which were cultured at 2 weeks post-challenge developed no plaques. By 5 weeks post-challenge, however, a significant level of plaque development (P < .01, 3.28, 28 df) was observed in unimmunized chicks followed by a further significant increase (P < .01, 3.43, 15 df) at 7 weeks post-challenge. Immunized chicks, however, failed to produce any significant plaque development through 7 weeks post-challenge.

Cultures from SPAFAS chicks demonstrated a significantly higher incidence of plaque development in unimmunized _versus_ immunized chicks at 2 weeks ($P < .01$, 3.28, 58 df), 5 weeks ($P < .01$, 5.62, 20 df) and 7 weeks ($P < .01$, 3.34, 34 df) post-MDV challenge. No significant plaque development occurred in immunized SPAFAS chicks throughout the experimental period.

Comparisons of lymphoma development between treatment groups presents a similar protection profile. Immunized P line chicks showed a higher incidence of lymphoma development than N line chicks, broilers of SPAFAS through 5 weeks post-MDV challenge, although all lines were significantly ($P < 0.01$) protected against challenge when compared with non-immunized, MDV-challenged chicks.

At 2 weeks post-challenge, no significant difference was seen between immunized and unimmunized chicks from any line, after MDV challenge. However, by 5 weeks post-challenge, lymphoma development was observed in 75 - 100% of unimmunized chicks, while only P line chicks which had been immunized developed a significant percentage of lymphomas after MDV challenge. Nevertheless, immunized P line chicks showed a 55 % reduction in lymphoma development over unimmunized chicks. By 7 weeks post-challenge of P line chicks, immunization no longer effected a reduction in tumor development, as 90 - 100% of all test chicks were seen to develop lymphomas. An increase in lymphoma development was seen in immunized N lines, broilers and SPAFAS at 7 weeks post-challenge as well, when compared with 5 week data. In the case of broilers and SPAFAS, immunization still resulted in at least a 50% reduction in lymphoma development at 7 weeks post-challenge. Lymphoma development in immunized N line chicks at 7 weeks was below 40% in both immunized and unimmunized chicks.

No virus was recovered from, nor tumor development observed in chicks which received no treatment or in chicks receiving only supernatant immunization.

## TABLE 7

Lymphoma development[a] in N and P line chicks immunized with JMV-1 culture supernatant and challenged with MDV.

P LINE

| Treatment | 2 wks | 5 wks | 7 wks |
|---|---|---|---|
| Supernatant + MDV | 2/10[b] | 5/12 | 10/10 |
| MDV | 3/12 | 9/10 | 7/7 |

N LINE

| Treatment | 2 wks | 5 wks | 7 wks |
|---|---|---|---|
| Supernatant + MDV | 1/15 | 1/15 | 2/12 |
| MDV | 2/10 | 11/14 | 6/14 |

[1]Day old chicks received immunizing dose of cell free JMV-1 culture supernatant or no treatment. At 15 days, 1/2 of each of these groups received 200-500 PFUs of Conn B strain of MDV. Assays conducted at 2, 5, and 7 weeks post-MDV challenge.

[b]Number of positive chicks/total chicks observed. Lymphoma developement was not observed in any chicks receiving no treatment or supernatant immunization only.

0244760

TABLE 8

Lymphoma development[a] in broiler and Specific-pathogen-
free chicks immunized with JMV-1 culture supernatant
and challenged with MDV.

BROILERS

| Treatment | 2 wks | 5 wks | 7 wks |
|---|---|---|---|
| Supernatant + MDV | 0/6[b] | 0/5 | 1/6 |
| MDV | 0/6 | 5/5 | 6/6 |

SPAFAS

| Treatment | 2 wks | 5 wks | 7 wks |
|---|---|---|---|
| Supernatant + MDV | 1/13 | 1/7 | 2/8 |
| MDV | 1/10 | 6/8 | 5/6 |

[1]Day old chicks received immunizing dose of cell free
JMV-1 culture supernatant or no treatment. At 15 days,
1/2 of each of these groups received 200-500 PFUs of
Conn B strain of MDV. Assays conducted at 2, 5, and 7
weeks post-MDV challenge.

[b]Number of positive chicks/total chicks observed.
Lymphoma development was not observed in any chicks
receiving no treatment or supernatant immunization
only.

EXAMPLE 2

Assays of Cell Mediated Immunity.

A. DTH assay of cell mediated immunity. To evaluate the effects of immunization with JMV-1 antigens and subsequent MDV exposure on the delayed type hypersensitivity (DTH) response, the experimental birds were divided into 4 groups, as previously described, of up to 25 birds per group. DTH assays in JMV-1 immunized N and P line chicks demonstrated that this cell-mediated response is not preserved in immunized or non-immunized N line chicks after MDV challenge (Table 9) but is preserved in P line chicks ($P < 0.05$). The overall DTH response of P line chicks appears to be significantly less than the N line response, however this is a reflection of a higher differential response in control footpads for P line chicks than for those of the other lines tested.

Neither S nor K line chicks that were immunized with JMV-1 cells at day-of-age and subsequently challenged with MDV showed any statistically significant DTH response above that of unimmunized control chicks.

## TABLE 9

DTH index[1] of chicks immunized with JMV-1 cells and challenged with MDV.

|  | Treatment | | | |
| Line of Chicken | N.T. | JMV-1 only | JMV-1+MDV | MDV only |
| N Line | 8.40[a] | 8.56[a] | 2.90[b] | 2.97[b] |
| P Line | 0.78[a] | 0.93[a] | 0.74[a] | -0.13[b] |
| K Line | 4.70[a] | 5.29[a] | 5.56[a] | 2.80[a] |
| S Line | 2.52[a] | 2.73[a] | 1.31[a] | 1.77[a] |

[a]Different superscripts indicate statistically significant (P < 0.05) differences between means indices within lines.

$$^{1}DTH\ Index = \frac{\text{degree of swelling in test footpad} - \text{degree of swelling in control footpad}}{\text{difference in swelling in control footpads}}$$

------------------------------------------------------------

B. $^{51}Cr$ release assay. Spleen cells from N and P line, SPAFAS and broiler chicks were assayed for immune responsiveness against the non-productive cell line, JMV-1 and the productive cell line, MSB-1 targets cells at 2, 5, and 7 weeks post--MDV challenge. In addition, assays were conducted at 3, 6, 8, and 11 days p.i. against JMV-1 and MSB-1 as well as against MDV-infected kidney cell monolayers exhibiting approximately 50 plaque-forming units (PFUs) per well of 2 X $10^6$ cells. In 6 hr. assays, all treatment groups demonstrated no significant responsiveness when compared with no treat-

0244760

ment groups. The specific release (SR) varied between -9.47 and 8.03 (Tables 10, 11 & 12).

C. Plaque inhibition assay. Plaque inhibition assays were conducted to determine if a cell-mediated response against MDV antigens was elicited in adult chickens which were hyperimmunized with cell-free JMV-1 supernatant (Table 13). When PBLs from either supernatant immunized or unimmunized K line hatchmates were used as effector cells against PBL targets from MDV-infected K line chickens, a significant degree (P < 0.0001) of plaque inhibition in K line kidney cells was noted compared with plaque development in control cultures containing only PBL targets overlaid onto K line kidneys. However, there was no significant difference in the amount of plaque inhibition elicited by PBLs from supernatant-immunized chickens as compared with inhibition elicited by PBLs from unimmunized hatchmates. Neither was there any significant reduction in plaque inhibition correlated.

## TABLE 10

### $^{51}$Cr Release Assay of Cell-Mediated Immunity[a].

#### P LINE

| Treatment | Target | 2 wks | 5 wks | 7 wks |
|---|---|---|---|---|
| Supernatant + MDV | JMV-1 | 0.00[b] | 4.02 | 0.00 |
| | MSB-1 | 4.57 | 1.15 | 0.00 |
| MDV | JMV-1 | 0.66 | 3.15 | 0.00 |
| | MSB-1 | 2.87 | 4.00 | 0.00 |
| supernat | JMV-1 | 8.03 | 0.00 | 2.52 |
| | MSB-1 | 5.68 | 0.30 | 0.02 |

#### N LINE

| Treatment | Target | 2 wks | 5 wks | 7 wks |
|---|---|---|---|---|
| Supernatant + MDV | JMV-1 | 4.07 | 0.09 | 5.20 |
| | MSB-1 | 5.03 | 3.29 | 1.22 |
| MDV | JMV-1 | 2.30 | 3.00 | 4.05 |
| | MSB-1 | 1.10 | 2.32 | 1.31 |
| supernat | JMV-1 | 0.00 | 2.76 | 0.00 |
| | MSB-1 | 1.23 | 3.45 | 0.00 |

[1]Day old chicks received immunizing dose of cell free JMV-1 culture supernatant or no treatment. At 15 days, 1/2 of each group received 200-500 PFUs of Conn B strain of MDV. Assays conducted at 2, 5, and 7 weeks post-MDV challenge.

[b]Percent cell lysis was determined as the amount of $^{51}$Cr released in presence of spleen cells from treated chicks minus amount of $^{51}$Cr released in the presence of spleen cells from no treatment chicks divided by maximum $^{51}$Cr release minus amount of $^{51}$Cr released in presence of spleen cells from no treatment chicks, all times 100.

## TABLE 11

### $^{51}Cr$ release Assay of cell mediated Immunity[a].

#### BROILERS

| Treatment | Target | 2 wks | 5 wks | 7 wks |
|---|---|---|---|---|
| Supernat + MDV | JMV-1 | -1.50 | 0.45 | 0.04 |
| | MSB-1 | 1.6 | 2.70 | 2.35 |
| MDV | JMV-1 | -1.83 | 0.19 | 0.19 |
| | MSB-1 | 1.27 | 1.90 | 1.82 |
| supernat | JMV-1 | 0.00 | 2.19 | 0.00 |
| | MSB-1 | 0.09 | -2.07 | 1.48 |

#### SPAFAS

| Treatment | Target | 2 wks | 5 wks | 7 wks |
|---|---|---|---|---|
| Supernat + MDV | JMV-1 | 1.05 | 0.15 | -1.36 |
| | MSB-1 | | -0.36 | -8.14 |
| MDV | JMV-1 | 0.66 | 0.18 | 1.30 |
| | MSB-1 | | -0.77 | -11.19 |
| supernat | JMV-1 | -0.53 | -1.35 | -1.75 |
| | MSB-1 | | -1.08 | -9.47 |

[1] Day old chicks received immunizing dose of cell free JMV-1 culture supernatant or no treatment. At 15 days, 1/2 of each group received 200-500 PFUs of Conn B strain of MDV. Assays conducted at 2, 5, and 7 weeks post-MDV challenge.

[b] Percent cell lysis was determined as the amount of $^{51}Cr$ released in presence of spleen cells from treated chicks minus amount of $^{51}Cr$ released in the presence of spleen cells from no treatment chicks divided by maximum $^{51}Cr$ release minus amount of $^{51}Cr$ released in presence of spleen cells from no treatment chicks, all times 100.

## TABLE 12

$^{51}$Cr release assay of cell mediated immunity, 3 to 11 days post-MDV challenge.

| Target Cells | Day p.i. | & Specific Release | | |
| --- | --- | --- | --- | --- |
| | | N.T. | MDV ± SUP | MDV only |
| JMV-1 | 3 | -3.17 | -0.86 | -1.78 |
| | 6 | 0.36 | 0.63 | 0.14 |
| | 8 | -0.90 | 0.35 | 0.08 |
| | 11 | 0.02 | 0.37 | 0.52 |
| MSB-1 | 3 | 0.27 | 1.36 | 1.48 |
| | 6 | 0.02 | -0.01 | -0.07 |
| | 8 | 1.27 | 1.09 | 1.17 |
| | 11 | 1.05 | 1.03 | -0.63 |
| K Line infect. kidney | 3 | 4.09 | 4.62 | 6.49 |
| | 6 | -0.62 | -2.14 | -2.03 |
| | 8 | 0.07 | -0.87 | -2.84 |
| | 11 | 1.05 | 1.03 | -0.63 |
| K Line kidney normal | avg. | -2.15 | -1.15 | -1.15 |

[b]Percent cell lysis was determined as the amount of $^{51}$Cr released in presence of spleen cells from treated chicks minus amount of $^{51}$Cr released in the presence of spleen cells from no treatment chicks divided by maximum $^{51}$Cr release minus amount of $^{51}$Cr released in presence of spleen cells from no treatment chicks, all times 100.

## TABLE 13

Plaque inhibition assay.

| Effector Cells | Dilution of Effector Cells | | |
|---|---|---|---|
| | $1 \times 10^7$ | $1 \times 10^6$ | $1 \times 10^5$ |
| Supernat. immunized | [a]5.30 ± 4.8 | 13.57 ± 13.07 | 13.86 ± 13.3 |
| Non-immunized | 7.31 ± 6.14 | 19.38 ± 11.84 | 17.77 ± 8.69 |
| Control | 51.86 ± 18.60 | | |

[a]Average number of plaques which developed in K line kidney monolayers, ± standard error of the mean. Targets are PBLS from K Line MDV-infected chicks.

## EXAMPLE 3

Assays of Humoral Immunity.

A.    Virus neutralization assays.    In vitro assays of virus neutralization with sera from the 4 previously described treatment groups indicated that chicks that were immunized with cell-free JMV-1 culture supernatant prior to MDV challenge, without exception, developed a higher neutralizing antibody titer than non-immunized, MDV challenged chicks.  Table 14 shows the dilution of antisera required to provide a 90% reduction in MDV- specific plaque development for SPAFAS chicks from 2 to 7 weeks p.i., and P and N line chicks at 5 and 7 weeks p.i.   For comparison, the average number of plaques that developed from kidney cell cultures established from these chicks as well as the incidence of lymphoma development is included in table 14.  At 2 weeks p.i., no detectable neutralizing antibody had developed in SPAFAS·chicks in either immunized or unimmunized groups but MDV-specific plaque development and lymphoma development was noted in unimmunized chicks.   At 5 weeks p.i., SPAFAS, P line and N line chicks all showed antibody titers above 40 to be developing in chicks which had  been immunized at day-of-age with cell-free JMV-1 culture supernatant, 2 weeks prior to MDV challenge. However, only one N line chick and no SPAFAS of P Line chicks which were not immunized prior to challenge developed a detectable antibody titer at 5 weeks p.i. At this time p.i., virus recovery from kidney cell cultures was high in all unimmunized, MDV challenged SPAFAS and P line chicks and in one of two N line chicks.  Kidneys which were cultured from the other N line chick which had developed a VN titer of 80, showed little plaque development. At 7 weeks p.i., antibody titers remained above 40 in all lines of chicks receiving supernatant immunization prior to MDV challenge. Virus recovery remained low and no lymphoma development was noted with the exception of one SPAFAS chick that had an antibody titer of 40. N line chicks receiving no immunization prior to MDV challenge maintained an antibody titer of 40 with a concomitant low degree of virus recovery and no observable tumor development. P line and SPAFAS chicks from this treatment group did not develop a detectable titer, however. Virus recovery remained high and tumor development was evident.

In no instance did any chicks develop a virus neutralizing antibody titer in the absence of MDV challenge. All assays were conducted using MDV convalescent sera as a positive control and serum from day-old SPAFAS chicks as a negative control.

B. <u>Maternal antibody assay</u>. After pretesting adult K line dams by AGID and kidney cell culture for the presence of MDV, dams were hyperimmunized with cell-free JMV-1 culture supernatant over a 14 week period. Progeny from these dams, as well as from unimmunized K line hatchmates, were challenged at day-of-age with 500 PFUs of Conn B MDV. At 2 weeks p.i., chicks were exsanguinated by cardiac puncture, examined for gross tumor development and the kidneys were cultured and observed for the development of MDV-specific CPE. No significant difference in plaque development was noted between kidney cell cultures from the progeny of supernatant immunized dams and unimmunized dams (Table 15).

## TABLE 14

### Marek's disease virus neutralization assay.[a]

| Line of Chick | Weeks p.i. | Sup + MDV | Avg. # Plaques[b] | Tumors[c] | MDV only | Avg. # Plaques | Tumors |
|---|---|---|---|---|---|---|---|
| SPAFAS | 2 | < 20 | 0.0 | – | <20 | 11.6 | + |
|  |  | < 20 | 0.0 | – | <20 | 3.6 | + |
|  | 5 | > 640 | 0.0 | – | <20 | 6.7 | + |
|  |  | 320 | 0.0 | – | <20 | 17.7 | + |
|  |  | 80 | 0.0 | – | <20 | 18.3 | + |
|  |  | 40 | 0.0 | – | <20 | 12.0 | + |
|  | 7 | 160 | 0.0 | – | <20 | 10.0 | + |
|  |  | 40 | 5.0 | + | <20 | 24.0 | + |
|  |  | 160 | 2.3 | – | <20 | >200.0 | + |
|  |  | > 640 | 0.0 | – | <20 | >200.0 | + |

(+) control: >640.  (−) control: <20.  Sup. only: <20.
No trmt: <20.

| Line of Chick | Weeks p.i. | Sup + MDV | Avg. # Plaques[b] | Tumors[c] | MDV only | Avg. # Plaques | Tumors |
|---|---|---|---|---|---|---|---|
| P Line | 5 | 80 | 4.7 | – | <20 | 70.6 | + |
|  |  | 160 | 1.0 | – | <20 | 48.0 | + |
|  | 7 | 40 | 0.6 | – | <20 | 72.0 | + |

(+) control: >640.  (−) control: <20.  Sup. only: <20.
No trmt: < 20.

| Line of Chick | Weeks p.i. | Sup + MDV | Avg. # Plaques[b] | Tumors[c] | MDV only | Avg. # Plaques | Tumors |
|---|---|---|---|---|---|---|---|
| N Line | 5 | 320 | 0.0 | – | < 20 | >200.0 | + |
|  |  | 160 | 0.6 | – | 80 | 0.6 | + |
|  | 7 | 80 | 0.6 | – | 40 | 0.6 | – |
|  |  | 320 | 0.0 | – | 40 | 0.0 | – |

(+) control: 40.  (−) control: <5.  Sup. only: <5.
No trmt: <20.

[a] Serum neutralizing titer (SN) = reciprocal of the highest dilution giving 90% Plaque reduction.

[b] Average number of plaques from triplicated kidney cell cultures from test chicks.

[c] (+) and (−) indicate the presence or absence of gross lymphoma development in test chicks.

## TABLE 15

### Plaque development in kidney cell cultures from progeny of supernatant immunized or unimmunized dams.

#### Plaque development at 2 wks p.i.[a]

| Progeny of immunized dams | Progeny of unimmunized dams |
|---|---|
| 14.58 ± 13.76 | 20.05 ± 25.40 |

[a]Plaque counts are an average of 20 kidney cell cultures per group grown in triplicate cultures of $2 \times 10^6$ cells.

---

C. **Agid profiles.** Agar gel immunodiffusion profiles of N and P line chicks from all treatment groups are displayed in Table 16. In no instance, did a precipitating antibody response develop in the absence of MDV challenge. No significant difference is seen in the number of chicks which are AGID positive versus AGID negative in MDV-challenged treatment groups.

## TABLE 16

**Agar gel immunodiffusion profile of N and P line, immunized and unimmunized chicks at 2, 5, and 7 wks post-MDV challenge.**

| Treatment | | 2 wks (+) | 2 wks (−) | 5 wks (+) | 5 wks (−) | 7 wks (+) | 7 wks (−) |
|---|---|---|---|---|---|---|---|
| Sup. + MDV | N Line | 2[a] | 4 | 6 | 0 | 4 | 0 |
| | P Line | 3 | 3 | 3 | 3 | 6 | 0 |
| MDV only | N Line | 1 | 5 | 6 | 0 | 4 | 2 |
| | P Line | 1 | 3 | 4 | 0 | | |

[a]Number of chicks positive or negative for AGID. All sera from no treatment and supernatant immunized only chicks were negative.

## EXAMPLE 4

### Efficacy of the JMV-1 supernatant vaccine

The efficacy of the JMV-1 supernatant vaccine was compared to that of cell-associated HVT vaccine and measured by challenge of vaccinated birds at 7 and 14 days post-vaccination using the JMV challenge agent.

The birds used were hatched 45 fertile eggs obtained from Spafas, Inc., SPF, Illinois. Lyophilized JMV-1 supernatant vaccine designated 7/10 was prepared using cells with a cell count of $3.3 \times 10^6$ per ml. Lyophilized JMV-1 supernatant vaccine designated 7/18 was prepared using cells with a cell count of $5.2 \times 10^6$ per ml, and lyophilized JMV-1 supernatant vaccine designated 7/23 was prepared using cells with a cell count of $3.8 \times 10^6$ per ml.

Groups of 40 birds each were intraperitoneally (IP) vaccinated with $1 \times 10^6$ cell equivalents of JMV-1 supernatant vaccine contained in 0.2 ml bird doses for 7/18 and 7/23 and 0.3 ml bird dose for 7/10. All vaccines were reconstituted with sterile Marek's diluent. The cell-associated HVT vaccine was given subcutaneously (SQ) in the nape of the neck to 20 hatchmate chicks using a 0.2 ml dose. Three (3) groups of 20 hatchmate chicks each were placed into isolation units. Two (2) groups of birds served as nonvaccinated challenge controls. The third group was placed as nonvaccinated, nonchallenged controls. The challenge agent was passed twice in 1-day-old chicks. The agent was titered in 1- and 2-week-old chicks. The titers at these respective ages were $10^{4.7}$ $CID_{50}$ per 0.2 ml and $10^{4.2}$ $CID_{50}$ per 0.2 ml. The JMV was diluted to contain approximately 250 $CID_{50}$ per 0.2 ml.

At seven (7) days post-vaccination (PV), 20 birds from each vaccinate group and 20 nonvaccinated controls were IP challenged with the diluted challenge using a 0.2 ml challenge dose. At fourteen (14) days after vaccination, the remaining vaccinates (20 per vaccine) and 20 nonvaccinated challenge controls were IP challenged with 250 $CID_{50}$ per 0.2 ml dose. The challenge birds were observed for mortality due to the JMV challenge from 3 to 14 days post-challenge The test was terminated 14 days after the second challenge. Each challenge was considered valid if at least 80% of its controls died. The test was considered valid if 90% of the HVT birds at each challenge survived.

Ninety-five (95) percent of the 7-day post-vaccination (PV) challenge control died. The mortality rate in the 14-day PV challenge controls was 89%. The HVT vaccine gave 90 and 100% challenge protection at the 7- and 14-day challenges, respectively. The vaccine identified as 7/18 gave 63% protection from the 7-day challenge and 55% from the 14-day PV challenge. Vaccination with the 7/23 vaccine resulted in 65% protection from the 7-day challenge and 53% from the 14-day challenge. Vaccination with the 7/10 vaccine resulted in 55% protection from the 7-day challenge and 45% protection from the 14-day challenge. In an earlier study vaccination with a JMV-1 supernatant vaccine prepared from cells grown until $1 \times 10^7$ per ml. was achieved after which the supernatant was removed and frozen, resulted in 45% protection from the 7-day challenge and 30% protection from the 14-day challenge.

- 56 -

0244760

TABLE 17

Results of JMV Challenge of Chicks Vaccinated With
JMV-1 Supernatant Vaccines

| Vaccine[a] | Challenge Protection | | | |
|---|---|---|---|---|
| | 7-Day Challenge[b] | | 14-Day Challenge[b] | |
| | # Live | Total % Protected | # Live | Total % Protected |
| HVT | 18/20 | 90 | 20/20 | 100 |
| 7/10 JMV-1 | 11/20 | 55 | 9/20 | 45 |
| 7/18 JMV-1 | 12/19 | 63 | 11/20 | 55 |
| 7/23 JMV-1 | 13/20 | 65 | 10/19 | 53 |
| Nonvaccinated, Challenge Controls | 1/20 | 5 | 2/19 | 11 |
| Nonvaccinated, Nonchallenged Controls | 20/20 | 100 | 20/20 | 100 |

a. HVT vaccine was given SQ. The JMV-1 vaccines were given IP.

b. All challenges were IP with JMV-1 CP2 with $10^{2.4}$ $CID_{50}$ per chick.

## EXAMPLE 5

### Efficiacy of the JMV-1 supernatant vaccine

Groups of 40 one-day-old SPF and broiler chickens may be SC vaccinated with the following MD vaccines.

1. HVT
2. JMV-1 SUPERNATANT
3. HVT + SB-1
4 HVT + SUPERNATANT

Twenty (20) vaccinates from each vaccine group and 20 nonvaccinated challenge controls are challenged at 7 days post-vaccination with the very virulent MD strain RBIB and observed for 56 days at which time the survivors are necropsied for tumors. Fourteen (14) days after vaccination, the remaining vaccinates and 20 nonvaccinated challenge controls are challenged as above.

## EXAMPLE 6

### Formulation and use of the JMV-1 supernatant for protection of chickens against Marek's disease

A composition for immunization of chickens against Marek's disease may be prepared from the JMV-1 supernatant and a suitable carrier. The supernatant vaccine is efficacious after lyophilization. Therefore, a lyophilized vaccine may be prepared. Also, a combination of the HVT and supernatant vaccine may be utilized. One suitable carrier for the vaccine is about 5% Arlacel A, 94% Drakeol 6-VR and 1% Tween 20. The vaccine may be administered to chickens of any age by injection, preferably intraperitoneally or subcutaneously. The vaccinated birds will be protected against disease, depressed performance or death caused by Marek's disease.

## References

1. Akiyama, Y., and Kato, S. (1974), Two lymphoblastoid lines from Marek's disease, Biken J. 17:105-116.

2. Calnek, B.W., Adlinger, H.K., and Kahn, D.E. (1970), Feather follicle epithelium: a source of enveloped and infectious cell-free herpesvirus from Marek's disease, Avian Dis. 14:219-233.

3. Calnek, B.W., Carlisle, J.C., Fabricant, J., Murthy, K.K., and Schat, K.A. (1979), Comparative pathogenesis studies with oncogenic and non-oncogenic Marek's disease viruses and turkey herpesvirus, Am. J. Vet. Res. 40:541-548.

4. Calnek, B.W., Schat, K.A., Fabricant, J., (1980), Modification of Marek's disease pathogenesis is by in ovo infection or prior vaccination. In, Viruses in naturally occurring cancers, Essex, Todaro and zur Hansen (eds.), Cold Spring Harbor Conferences on Cell Proliferation, 7:185-197.

5. Chubb, R.C., and Churchill, A.E., (1968), Precipitating antibodies associated with Marek's disease, Vet. Rec. 83:4-7.

6. Churchill, A.E., Payne, L.N., and Chubb, R.C., (1969), Immunization against Marek's disease using a live attenuated vaccine, Nature 211:744-747.

7. Hahn, E.C., Ramos, L., and Kenyon, A.J. (1977), Lymphoproliferative diseases of fowl: JM-V Leuke- mic lymphoblasts in cell culture, J. Natl. Cancer Inst. 59:267-271.

8.  Hong, C.C., and Sevoian, M., (1971), Interferon production and host resistance to type II (Marek's) leukosis virus (JM strain), Appl. Microbiol. 22:818-820.

9.  Hong, C.C., (1973), Studies on the antigen and antibody in susceptible (JM-P line) and resistant (JM-N line) chickens infected with JM-V leukosis strain, Ph.D. Disertation, University of Massachusetts, Amherst, Massachusetts.

10. Hong, C.C., and Sevoian, M., (1974) Serum neutralizing antibody development and host resistance in chickens exposed to Marek's disease infection, Poult. Sci. 53:1110-1113.

11. Hong, C.C., and Sevoian, M., (1974), The comparative influence of passive antibodies in early exposure to JM virus of progeny chicks, Avian Dis. 18:305-317.

12. Hutt, F.B., and Cole, R.K., (1947), Genetic control of lymphomatosis in the fowl, Science 106:379-384.

13. Jakowski, R.M., Frederickson, T.N., Chomiak, T.W., and Lugeinbuhl, R.E., (1970), Hematopoietic destruction in Marek's disease, Avian Dis. 14:374-380.

14. Kaaden, O.R., Dietzshold, B., Uberschar, S.,

(1974), Vaccination against Marek's disease: immunizing effect of purified turkey herpesvirus and cellular membranes from infected cells, Med. Microbiol. Immunol. 159:261-269.

15. Kawamura, H., King, D.J., Jr., and Anderson, D.P., (1969), A herpesvirus isolated from kidney cell cultures of normal turkeys., Avian Dis. 13:853-863.

16. Keller, L.H., (1981), Studies of Histocompatibility and Immune competence of chickens selected for resistance and susceptibility to Marek's disease, M.S. Thesis, University of Massachusetts, Amherst, MA.

17. Keller, L.H., and Sevoian M., (1983), Studies of histocompatibility and immune response of chickens selected for resistance and susceptibility to Marek's disease, Avian Dis. 27:7-20.

18. Landgraf, H., and Vielitz, E., (1978), Virksankeitsprufungen mit PHV-, MHV-und JMV-vakzinen gegen die Marek'sche Krankheit, Avian Path. 7:193-202.

19. Lesnik, F., and Ross, L.J.N., (1975), Immunization against Marek's disease using Marek's disease virus-specific antigens free from infectious virus, Int. J. Cancer 16:153-163.

20. Lowry, O.H., Rosebrough, N.J., Farr, A.L., and Randall, R.J., (1951), Protein measurement with

-62-

the folin phenol reagent, J. Biol. Chem. 193:265-275.

21.  Munch, D.C., Hohlstein (Keller), L., and Sevoian, M., (1978), In vitro establishment of Marek's disease herpesvirus-transformed productive and non-productive lymphoblastoid cell lines. Inf. & Immun. 20:213-318.

22.  Munch, D., and Sevoian, M., (1977), Establishment of a non-producer T lymphoblastoid cell line in vitro from JMV lymphoblastic leukemia, Presented at the Third International Conference on Oncogenesis and Herpesvirus, Cambridge, MA, July 26-30.

23.  Munch, D., and Sevoian, M., (1980), Growth and characterization of, and immunological response of chickens to, a cell line established from JMV lymphoblastic leukemia, Avian Dis. 24:23-36.

24.  Nazerian, K., Stevens, E.A., Sharma, J.M., Lee, L.F., Gailitis, M., and Witter, R.L., (1977), A non-producer T lympholastoid cell line from Marek's disease transplantable tumor (JMV), Avian Dis. 21:69-76.

25.  Nazerian, K., and Witter, R.L., (1983), Immunization against Marek's disease transplantable cell lines, Avian Dis. 28:160-167.

26.  Okazaki, W., Purchase, H.G., and Burmester B.R., (1970), Protection against Marek's disease by vaccination with a herpesvirus of turkeys, Avian Dis. 14:113-129.

27. Okazaki, W., Purchase, H.G., and Noll, L., (1970), Effect of different conditions on precipitation in agar between Marek's disease antigen and antibody, Avian Dis. 14:532-537.

28. Pazderka, F., Longenecker, B.M., Law, G.R.J., Stone, H.A., and Ruth, R.F., (1975), Review: The major histocompatibility complex of the chicken, Immunogenetics, 2:101-130.

29. Powell, P.C., (1975), Immunity to Marek's disease induced by glutaraldehyde treated cells of Marek's disease lymphoblastoid cell lines, Nature 257:684-685.

30. Powell, P.C., (1978), Protection against the JMV Marek's disease-derived transplantable tumour by Marek's disease virus-specific antigens, Avian Path 7:305-309.

31. Powell, P.C., and Rowell, J.G., (1979), Dissociation of antiviral and antitumor immunity in resistance to Marek's disease, J. Natl. Cancer Inst. 59:919-924.

32. Powell, P.C., Payne, L.N., Frazier, J.A., and Rennie, M., (1974), T lymphoblastoid cell lines from Marek's disease lymphomas, Nature 25:79-80.

33. Reed, L.J., and Meunch, H., (1938), Simple method of estimating 50% endpoints, Am. J. Hygiene 27:493-497.

34. Rispens, B.H., van Holten, H., Mastenbrock, N., Maas, H.J.L., and Schat, K.A., (1972), Control of Marek's disease in the Netherlands, I. Isolation of an avirulent Marek's disease virus (strain CVI-988) and its use in laboratory vaccination trials, Avian Dis. 16:108-125.

35. Schierman, L.W., Theis, G.A., and McBride, R.A., (1977), Preservation of T cell mediated immune response in Marek's disease virus infected chickens by vaccination with a related virus, J. Immunol. 110:1497-1499.

36. Sevoian, M., (1962), Avian lymphomatosis, I. Experimental reproduction of neural and visceral forms, Vet. Med. 57:560-561.

37. Sevoian, M., (1967), Immunity studies in chickens inoculated with a virulent type II avian leukosis strain (JM-V), Proc. 39th Northeastern Conference on Avian Disease, State University of. New York, Stony Brook, June 19-21.

38. Shieh, H.K., and Sevoian, M., (1974), Antibody response of susceptible and resistant chickens (type II leukosis) infected with JM and JM-V leukosis strains and herpesvirus of turkeys (HVT), Avian Dis. 18:318-326.

39. Shieh, H.K. and Sevoian, M., (1975), Antibody response of genetically susceptible and resistant chickens to cell-free and attenuated JM-V leukosis strain and its influence on early type II (Marek's) leukosis infection, Poult. Sci. _54_:69-77.

40. Shieh, H.K., and Sevoian, M., (1978), The influence of humoral and cellular immune system on chickens with Marek's disease, J. Chinese Vet. Sci. _4_:3-13.

41. Stephens, E.A., Witter, R.L., Lee, L.F., Sharma, J.M., Nazerian, K., and Longenecker, B.M., (1976), Characteristics of JMV Marek's disease tumor: A nonproductively infected transplantable cell lacking in rescuable virus, J. Natl. Can. Inst. _57_:865-872.

42. von Bulow, V., and Schmid, D.O., (1979), Antigenic characteristics of Marek's disease tumor cells, Avian Path. _8_:265-277.

43. Witter, R.L., and Lee, L.F., (1984), Polyvalent Marek's disease vaccines: Safety, efficacy and protective synergism in chickens with maternal antibodies, Avian Path. _13_:75-92.

44. Witter, R.L., Nazerian, K., Purchase, H.G., and Burgoyne, G.H., (1970), Isolation from turkeys of a cell-associated herpesvirus antigenically related to Marek's disease virus, Am. J. Vet. Res. _31_:525-538.

C l a i m s

1. A cell free supernatant of a culture of whole, live cells of an attenuated lymphoblastoid cell line which provides protection to an animal against infection by a herpesvirus or a serologically related strain thereof.

2. The supernatant of claim 1, wherein the herpesvirus is an avian herpesvirus.

3. The supernatant of claim 2, wherein the herpesvirus is Marek's disease virus.

4. The supernatant of claim 1, wherein the strain is serologically related to Marek's disease virus.

5. The supernatant of claim 4, wherein the strain is an attenuated lymphoblastoid cell line designated JMV-1 (ATCC No. CRL 9093).

6. The supernatant of claim 1, wherein the animal is avian, e.g. a chicken.

7. A vaccine for conferring upon an animal active immunity against infection by a herpesvirus or a serologically related strain thereof which comprises per dose an effective immunizing amount of the cell free supernatant of anyone of claims 1 to 5 and a suitable carrier.

8. A vaccine for conferring upon a chicken active immunity against infection by Merek's disease virus or a serologically related strain thereof which comprises per dose an effective immunizing amount of a cell free supernatant of anyone of claims 3 to 5 and a suitable carrier.

9. A method of preparing the supernatant of claim 1 which comprises:

   a. centrifuging a culture of whole live cells of a herpesvirus or a serologically related strain thereof so as to separate the cells from the supernatant;

   b. treating the product of step (a) so as to remove the cells; and

   c. recovering the supernatant.

10. The supernatant of anyone of claims 1 to 5 for use in conferring upon a chicken active immunity against infection by Marek's disease virus or a serologically related strain thereof.

11. The vaccine of claim 8 for use in protecting a chicken against infection by Marek's disease virus or a serologically related strain thereof.

12. The vaccine of claim 7 for use in protecting an animal against infection by a herpes virus or a serologically related strain thereof.

13. An immunogenic agent derived from the cell free supernatant of claim 1.

14. A lyophilized supernatant of claim 1.

Figure 1